# EUROPÄISCHE PATENTSCHRIFT

(11) **EP 2 467 374 B1**
(45) Veröffentlichungstag und Bekanntmachung des Hinweises auf die Patenterteilung: **01.05.2013**
(21) Anmeldenummer: 10742759.3
(22) Anmeldetag: 05.08.2010
(51) Int. Cl.: C07D 401/12, C07D 307/66

(54) **NEUES VERFAHREN ZUR HERSTELLUNG VON 4-AMINOBUT-2-ENOLIDEN**
New method for manufacturing 4-aminobut-2-enolids
Nouveau procédé de fabrication de 4-aminobut-2-énoïque

(30) Priorität: 18.08.2009 EP 09168068; 21.08.2009 US 235878 P
(43) Veröffentlichungstag der Anmeldung: 27.06.2012
(73) Patentinhaber: Bayer CropScience AG, 40789 Monheim (DE)
(72) Erfinder: LUI, Norbert, 51519 Odenthal (DE); HEINRICH, Jens-Dietmar, 51399 Burscheid (DE)
(86) Internationale Anmeldenummer: PCT/EP2010/004800
(87) Internationale Veröffentlichungsnummer: WO 2011/020564

(56) Entgegenhaltungen:
- EP-A- 2 039 678
- EP-A- 2 042 496
- WO-A-2007/115644
- MOWAFAK Y. SHANDALA, MIKDAD T. AYOUB, MOYAYED J. MOHAMMAD: "Reaction of methyl tetronate with some amines. Synthesis of substituted 4-aminobut-2-enolides" J. HETEROCYCLIC CHEM., Bd. 21, 1984, Seiten 1753-1754, XP002553839
- DATABASE CA [Online] CHEMICAL ABSTRACTS SERVICE, COLUMBUS, OHIO, US; 1973, MAVROV, M. V. ET AL: "Nucleophilic reactivity of conjugated bromoallenes" XP002553840 gefunden im STN Database accession no. 1973:417999 & DOKL. VSES. KONF. KHIM. ATSETILENA, 4TH ( 1972 ), VOLUME 1, 461-7 FROM: REF. ZH., KHIM. 1973, ABSTR. NO. 3ZH141, 1973,

## Beschreibung

Die vorliegende Erfindung betrifft ein Verfahren zur Herstellung von 4-Amino-but-2-enoliden.

Bestimmte substituierte 4-Aminobut-2-enolid-Verbindungen sind als insektizid wirksame Verbindungen aus EP-A-0 539 588 und WO 2007/115644 bekannt. Sie können nach verschiedenen Methoden dargestellt werden.

So ist beispielsweise in Heterocycles Vol. 27, Nr. 8, Seite 1907 bis 1923 (1988) und in EP-A-0 539 588 beschrieben, dass Enaminocarbonylverbindungen (3) aus wasserfreier Tetronsäure (1) und einem Amin (2), wie in Schema 1 dargestellt, hergestellt werden können. Dieses Verfahren ist jedoch zur großtechnischen Herstellung von Enaminocarbonylverbindungen nicht gut geeignet, da die wasserfreie Tetronsäure (1) nicht kostengünstig herstellbar ist.

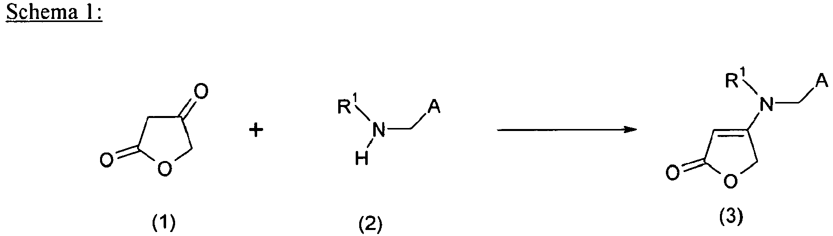

Bislang ist die Tetronsäure in größeren Mengen nicht käuflich erhältlich, so dass sie ausgehend von Acetessigester über Bromierung und anschließender Hydrierung (vgl. Synthetic Communication, 11(5), Seiten 385 bis 390 (1981)) zur Verwendung im oben beschriebenen Verfahren hergestellt werden muss. Die verhältnismäßig geringe Ausbeute an Tetronsäure (gewöhnlicherweise kleiner als 40%) und die Bedingung, dass Tetronsäure wasserfrei sein muss, verursacht hohe Kosten.

Ein weiteres Verfahren zur Herstellung von Tetronsäure ist in CH-PS 503 722 beschrieben. 4-Chloracetessigester wird mit einem aromatischen Amin zum 3-Arylaminocrotonlacton umgesetzt, wobei nach anschließender Behandlung mit Mineralsäure die Tetronsäure freigesetzt wird. Die Tetronsäure kann nur durch Hochvakuumdestillation isoliert werden, was für den großtechnischen Einsatz dieses Verfahrens nachteilig ist.

Gleichfalls beschreibt die EP-A-0 153 615 ein mehrstufiges Verfahren zur Herstellung von Tetronsäure das von 2,4-Dichloracetessigestern ausgeht, das für die großtechnische Herstellung schlecht geeignet ist. Diese Verfahren benötigt viele aufwändige Stufen und liefert die gewünschte Tetronsäure in einer vergleichsweise mäßigen Ausbeute von 65 %.

Ein weiteres Verfahren zur Herstellung von Tetronsäure ausgehend von Malonestern und Chloracetylchlorid ist aus J. Chem. Soc. Perkin Trans. 1 (1972), 9/10, 1225-1231 bekannt. Dieses Verfahren liefert die gewünschte Zielverbindung, jedoch mit einer Ausbeute von nur 43 %.

In Tetrahedron Letters, Nr. 31, Seiten 2683 und 2684 (1974) ist unter anderem die Herstellung von Tetronsäure beschrieben, die in Schema 2 wiedergegeben ist. Als Edukt wird dabei Acetylendicarbonsäuredimethylester eingesetzt.

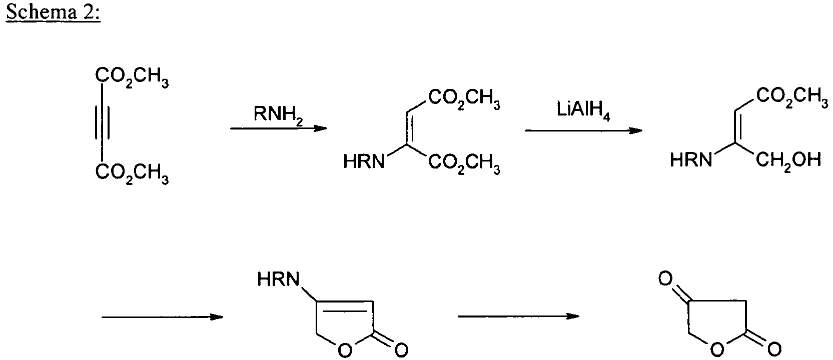

Nachteilig an diesem Verfahren ist die geringe Gesamtausbeute von nur 30 % sowie der Einsatz teurer Ausgangsstoffe, wie beispielsweise Lithiumaluminiumhydrid (LiAlH₄).

Ein Verfahren zur Herstellung von am Stickstoff substituierten 4-Amino-2(5H)furanonen, das von der Tetronsäure ausgeht, wird in Heterocycles, Vol. 27, Nr. 8, 1988, 1907-1923 beschrieben. In diesem Verfahren wird von einem 4-Chloracetessigester ausgegangen, der mit den entsprechenden Aminen umgesetzt wird, wobei die Reaktion zum Aminofuran in einem Schritt durchgeführt wird. Dabei wird das Amin mit Eisessig zu einer Lösung von 4-Chloracetessigester in Benzol gegeben und die resultierende Mischung wird mehrere Stunden unter Rückfluss erhitzt. Die Ausbeuten an 4-Methylamino-2(5H)-furanon in dieser Synthese betragen lediglich 40 %.

Ein weiteres Verfahren zur Herstellung von substituierten 4-Aminobut-2-enoliden ist von Mowafak et al. in J. Heterocyclic Chem., 21, 1753-1754 (1984) beschrieben. Dieses Verfahren geht von Methyltetronat aus, wobei durch Umsetzung mit Aminen die gewünschten Verbindungen hergestellt werden. Die Herstellung von Methyltetronat verläuft über eine mehrstufige Synthese in der trockene Lösungsmittel und teure Chemikalien, wie z.B. 4-Brom-3-methoxy-but-3-encarbonsäureester eingesetzt werden, so dass das Verfahren großtechnisch nicht vorteilhaft ist. Aus EP-A-0 123 095 ist ein Verfahren bekannt, in welchem Tetronsäureamid aus 3-Amino-4-acetoxycrotonsäureester hergestellt wird. Der Ausgangstoff 3-Amino-4-acetoxycrotonsäureester ist jedoch aufwändig und dadurch nur teuer herzustellen, so dass auch hier diese Synthese für die großtechnische Herstellung nicht geeignet ist.

In der WO 2007/115644 wird die Herstellung von speziellen 4-Aminobut-2-enoliden beschrieben. Beispielsweise von 4-[[(6-Chlorpyridin-3-yl)methyl](3,3-dichlorprop-2-en-1-yl)amino]furan-2(5H)-on durch Umsetzung von 4-[[(6-Chlorpyridin-3-yl)methyl]amino]furan-2(5H)-on mit 3-Brom-1,1-dichlorprop-1-en oder durch Umsetzung von 4-[(2-Fluorethyl)amino]furan-2(5H)-on mit 2-Chlor-5-chlormethyl-pyridin. Die Reaktionen werden vorzugsweise mit Hydriden des Lithiums oder Natriums durchgeführt. Diese Substrate sind auch hier teuer und aus Sicherheitsgründen nur schwer zu handhaben.

Ausgehend von diesem Stand der Technik stellt sich somit die Aufgabe, ein Verfahren zur Herstellung von 4-Aminobut-2-enolid-Verbindungen bereitzustellen, welches einfach und kostengünstig durchzuführen ist, so dass das Verfahren auch zur großtechnischen Herstellung von 4-Aminobut-2-enolid-Verbindungen verwendet werden kann und die 4-Aminobut-2-enolid-Verbindungen mit hoher Ausbeute und ausreichend hoher Reinheit liefert, so dass keine aufwändigen Aufreinigungsmethoden notwendig sind.

Es wurde nun ein Verfahren zur Herstellung von 4-Aminobut-2-enolid-Verbindungen gefunden, das die vorgenannten Nachteile vermeidet und das einfach und kostengünstig durchführbar ist, insbesondere weil die erfindungsgemäßen 4-Aminobut-2-enolid-Verbindungen mit guten Ausbeuten und in hoher Reinheit erhalten werden, so dass eine aufwändige Aufarbeitung bzw. Reinigung des unmittelbaren Reaktionsprodukts gewöhnlicherweise nicht erforderlich ist.

Gegenstand der Erfindung ist somit das unten beschriebene Verfahren zur Herstellung von 4-Aminobut-2-enolid-Verbindungen der Formel (I): 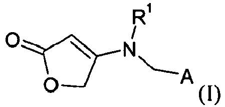 worin R¹ und A für die weiter unten definierten chemischen Gruppierungen stehen.

Das erfindungsgemäße Verfahren zur Herstellung von 4-Aminobut-2-enolid-Verbindungen der Formel (I) umfasst das Umsetzen einer 4-(Methylamino)furan-2(5H)-on-Verbindung der Formel (II) 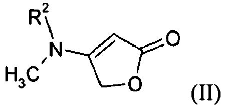 mit einem Amin der Formel (III) 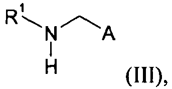 worin
- R¹: für Wasserstoff, C₁₋₁₂-Alkyl, C₁₋₂-Halogenalkyl, C₂₋₁₂-Alkenyl, C₂₋₁₂-Halogenalkenyl, C₂₋₆-Alkinyl, C₃₋₈-Cycloalkyl, C₃₋₈-CycloalkylC₁₋₆-alkyl, C₃₋₈-Halogencycloalkyl, C₁₋₁₂-Alkoxy, C₁₋₆-AlkyloxyC₁₋₆-alkyl, C₃₋₈-HalogencycloalkylC₁₋₆-alkyl oder ArylC₁₋₆-alkyl steht, bevorzugt steht R¹ für C₁₋₆-Alkyl, C₁₋₆-Halogenalkyl, C₂₋₆-Alkenyl, C₂₋₆-Halogenalkenyl, C₂₋₆-Alkinyl, C₃₋₈-Cycloalkyl, C₃₋₈-CycloalkylC₁₋₆-alkyl, C₃₋₈-Halogencycloalkyl, C₃₋₈-HalogencycloalkylC₁₋₆-alkyl oder C₁₋₆-AlkyloxyC₁₋₆-alkyl, besonders bevorzugt für Methyl, Ethyl, Propyl, Propylen, Vinyl, Allyl, Propargyl, Cyclopropyl, C₁₋₆-AlkyloxyC₁₋₆-alkyl, 2-Fluorethyl, 2,2-Difluorethyl oder 2-Fluorcyclopropyl, ganz besonders bevorzugt für Methyl, Ethyl, n-Propyl, n-Prop-2-enyl, n-Prop-2-inyl, Cyclopropyl, Methoxyethyl, 2-Fluorethyl oder 2,2-Difluorethyl;
- R²: für C₁₋₁₂-Alkyl, Aryl oder ArylC₁₋₆-alkyl steht, bevorzugt steht R² für C₁₋₆-Alkyl, Phenyl oder ArylC₁₋₆-alkyl, besonders bevorzugt für Methyl oder Ethyl; und
- A: für Pyrid-2-yl, Pyrid-4-yl oder Pyrid-3-yl steht, welches gegebenenfalls in 6-Position durch F, Cl, Br, CH₃, CF₃, oder OCF₃ substituiert ist, für Pyridazin-3-yl steht, welches gegebenenfalls in 6-Position durch Cl oder CH₃ substituiert ist, für Pyrazin-3-yl, 2-Chlorpyrazin-5-yl oder für gegebenenfalls in 2-Position durch Cl oder CH₃ substituiertes 1,3-Thiazol-5-yl, für Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, das gegebenenfalls durch F, Cl, Br, CN, NO₂, C₁₋₄-Alkyl, C₁₋₃-Alkylthio, oder C₁₋₃-Alkylsulfonyl substituiert ist wobei jeder der Reste C₁₋₄-Alkyl, C₁₋₃-Alkylthio und C₁₋₃-Alkylsulfonyl durch F und/oder Chlor substituiert sein können, oder für ein substituiertes Heterocyclyl der folgenden Formel steht 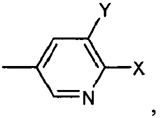
in der
- X: für Halogen, C₁₋₁₂-Alkyl oder C₁₋₁₂-Halogenalkyl steht und
- Y: für Halogen, C₁₋₁₂-Alkyl, C₁₋₁₂-Halogenalkyl, C₁₋₁₂-Halogenalkoxy, Azido oder CN steht;
bevorzugt steht A für ein substituiertes Heterocyclyl ausgewählt unter 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl oder 5-Difluormethyl-6-iod-pyrid-3-yl, besonders bevorzugt steht A für ein substituiertes Heterocyclyl ausgewählt unter 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl oder 5-Difluormethyl-6-chlor-pyrid-3-yl, ganz besonders bevorzugt steht A für ein substituiertes Heterocyclyl ausgewählt unter 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl,
gegebenenfalls in Gegenwart einer Brønstedt-Säure.

Überraschenderweise wurde gefunden, dass in der erfindungsgemäßen Reaktion bzw. Umsetzung der Austausch des Methyl-Alkyl-Aminrestes in der Verbindung der Formel (II) gegen das Amin der Formel (III) in sehr guten Ausbeuten erfolgt und dass die Tetronsäure unter den Reaktionsbedingungen nicht dimerisiert, obwohl eine solche Dimerisierung nach J. Chem. Soc. (1947) Seite 1365 zu erwarten war.

Die erfindungsgemäße Umsetzung kann ferner in Gegenwart von Lösungsmitteln (Verdünnungsmitteln) durchgeführt werden. Das Lösungsmittel wird vorzugsweise in einer solchen Menge eingesetzt, dass das Reaktionsgemisch während des ganzen Verfahrens gut rührbar bleibt. Als Lösungsmittel zur Durchführung des erfindungsgemäßen Verfahrens bzw. Umsetzung kommen alle unter den Reaktionsbedingungen inerten organischen Lösungsmittel in Frage. Unter Lösungsmittel werden erfindungsgemäß auch Gemische reiner Lösungsmittel verstanden.

Erfindungsgemäß geeignete Lösungsmittel sind insbesondere Halogenkohlenwasserstoffe, wie Chlorkohlenwasserstoffe (z.B. Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Ether (z.B. Ethylpropylether, Methyl-tert-butylether, *n*-Butylether, Anisol, Phenetol, Cyclohexylmethylether, Dimethylether, Diethylether, Dimethylglycol Diphenylether, Dipropylether, Diisopropylether, Di-*n*-butylether, Diisobutylether, Diisoamylether, Ethylenglycoldimethylether, Isopropylethylether, Tetrahydrofuran, Methyl-Tetrahydrofuran, Dioxan, Dichlordiethylether und Polyether des Ethylenoxids und/oder Propylenoxids), Nitrokohlenwasserstoffe (z.B. Nitromethan, Nitroethan, Nitropropan, Nitrobenzol, Chlornitrobenzol, o-Nitrotoluol), Nitrile (z.B. Acetonitril, Methylnitril, Propionitril, Butyronitril, Isobutyronitril, Benzonitril, Phenylnitril, m-Chlorbenzonitril) sowie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon, aliphatische, cycloaliphatische oder aromatische Kohlenwasserstoffe (z.B. Pentan, n-Hexan, n-Heptan, n-Oktan, Nonan, sogenannte "White Spirits" mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Xylol; Ester wie Methyl-, Ethyl-, Butyl-, Isobutylacetat, sowie Dimethyl-, Dibutyl-, Ethylencarbonat), Amide (z.B. Hexamethylenphosphorsäuretriamid, Formamid, N,N-Dimethyl-acetamid, *N*-Methyl-formamid, *N*,*N*-Dimethyl-formamid, *N,N*-Dipropyl-formamid, *N*,*N-*Dibutyl-formamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formylpiperidin, *N,N*'-1,4-Diformyl-piperazin), und aliphatische Alkohole (z.B. Methanol, Ethanol, n-Propanol und iso-Propanol und n-Butanol) oder Gemische davon.

Für das erfindungsgemäße Verfahren bzw. die Umsetzung werden als Lösungsmittel bevorzugt Dioxan, Butyronitril, Propionitril, Acetonitril, Butylacetat, DME, Toluol, Methyl-THF, Dichlorbenzol, Chlorbenzol, n-Heptan, iso-Butanol, n-Butanol, Ethanol, Methyl-tert.-butylether, Isopropylethylether und Mischungen davon verwendet.

In Abhängigkeit der verwendeten Ausgangsverbindungen kann das erfindungsgemäße Verfahren bzw. die Umsetzung in Substanz, d.h. ohne Zusatz an Lösungsmitteln, durchgeführt werden.

Erfindungsgemäß geeignete Brønstedt-Säuren sind grundsätzlich alle organischen und anorganischen Säuren. Erfindungsgemäß bevorzugte Brønstedt-Säuren sind Phosphorsäure (H₃PO₄), Schwefelsäure (H₂SO₄), Salzsäure (HCl), Bromwasserstoffsäure (HBr), Flusssäure (HF), Kaliumhydrogensulfat (KHSO₄), Trifluoressigsäure, Essigsäure, Methansulfonsäure und p-Toluolsulfonsäure. Erfindungsgemäß besonders bevorzugt sind Phosphorsäure, Schwefelsäure, Kaliumhydrogensulfat und Trifluoressigsäure.

Die Brønstedt-Säure kann sowohl in wasserfreier als auch in wasserhaltiger Form, beispielsweise als 85%-ige Phosphorsäure oder 37-%ige Salzsäure, vorliegen. Aus wirtschaftlichen Gründen ist es bevorzugt, die im Handel erhältlichen Säurekonzentration zu verwenden.

Das Verhältnis der eingesetzten Brønstedt-Säure zu dem Amin der Formel (III) kann variieren. Vorzugsweise liegt das Verhältnis von Brønstedt-Säure zum Amin der Formel (III) im Bereich von etwa 5 : 0,8 bis etwa 1: 1,5, insbesondere von etwa 3: 0,9 bis 1: 1,2, speziell von etwa 1,5:1 bis etwa 1: 1,1.

Das erfindungsgemäßen Verfahren kann im Allgemeinen im Vakuum, bei Normaldruck oder unter Überdruck durchgeführt werden.

Die angewendeten Temperaturen können in Abhängigkeit der verwendeten Ausgangsstoffe variieren. Die erfindungsgemäße Umsetzung bzw. das Verfahren kann bei Temperaturen im Bereich von etwa 20 °C bis etwa 200 °C, vorzugsweise bei Temperaturen im Bereich von etwa 20 °C bis etwa 150 °C durchgeführt werden.

Die Stöchiometrie der eingesetzten Verbindungen der Formel (II) und (III) kann in weiten Bereichen variieren. Das molare Verhältnis der Verbindung der Formel (II) zu dem eingesetzten Amin der Formel (III) kann etwa 1:0,5 bis etwa 1:10, insbesondere etwa 1:1 bis etwa 1:6, speziell etwa 1:1,05 bis etwa 1:2 betragen. Der Einsatz größerer Mengen an Verbindung der Formel (III) ist grundsätzlich möglich, jedoch aus wirtschaftlichen Gründen nachteilig.

Falls die Umsetzung in einem Lösungsmittel durchgeführt wird, kann das Lösungsmittel nach dem Reaktionsende durch Abdestillieren entfernt werden. Dieses kann unter Normaldruck oder erniedrigtem Druck bei Raumtemperatur oder erhöhten Temperaturen erfolgen.

Nach Ende der Reaktion können die entstandenen Ammoniumsalze durch Extraktion mit Wasser entfernt werden. Die Isolierung der gewünschten Verbindungen der Formel (I) kann durch übliche Methoden, insbesondere durch Kristallisation erfolgen.

4-(Methylamino)furan-2(5H)-on-Derivate der Formel (II) sind zum Teil bekannt und/oder können nach gängigen Methoden hergestellt werden.

Die Herstellung von Verbindung der Formel (II) in der R² für Methyl steht, ist beispielsweise in Heterocycles Vol. 27, 8, 1988, 1907-1923 beschrieben. Die Herstellung von Verbindungen der Formel (II), in denen R² für Wasserstoff steht, ist beispielsweise in WO 2009/036898 beschrieben.

Ein Syntheseweg für Verbindungen der Formel (II), in denen R² für H bzw. für erfindungsgemäßes Alkyl steht, ist in Schema 3 dargestellt. Ausgehend von 4-Chloracetoacetat (4) wird eine Verbindung der Formel (II) hergestellt, in der R² für H steht und die dann anschließend mit einem Alkylierungsmittel X-R^{2'} umgesetzt wird. R^{2'} steht dabei für eine erfindungsgemäße Alkylgruppe und X für eine geeignete Abgangsgruppe. Geeignete Abgangsgruppen sind solche, die bei den vorherrschenden Reaktionsbedingungen eine ausreichende Nucleofugizität aufweisen, wie beispielsweise Halogene (z.B. Cl, Br, oder Jod), Mesylat, Tosylat oder SO₂Me, insbesondere Cl, Br und Mesylat.

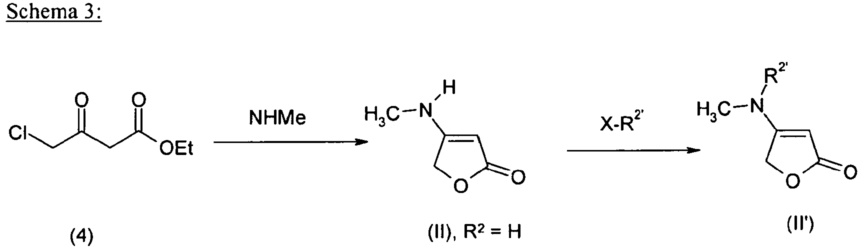

Unter dem Begriff "Alkyl", entweder in Alleinstellung oder aber in Kombination mit weiteren Begriffen, wie beispielsweise Halogenalkyl, Alkoxyalkyl, Cycloalkylalkyl, Halogencycloalkylalkyl und Arylalkyl, wird im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁₋₁₂-Alkylreste sind Methyl, Ethyl, n-Propyl, isoPropyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alkylresten sind C₁₋₆-Alkylreste besonders bevorzugt. Insbesondere bevorzugt sind C₁₋₄-Alkylreste, speziell Methyl und Ethyl.

Unter dem Begriff "Alkenyl" wird erfindungsgemäß ein linearer oder verzweigter C₂₋₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butandienyl, 1-Pentenyl, 2-Pentenyl, 3-Pentenyl, 4-Pentenyl, 1,3-Pentandienyl, 1-Hexenyl, 2-Hexenyl, 3-Hexenyl, 4-Hexenyl, 5-Hexenyl und 1,4-Hexandienyl, verstanden. Bevorzugt hiervon sind C₂₋₆-Alkenylreste und besonders bevorzugt sind C₂₋₄-Alkenylreste.

Unter dem Begriff "Alkinyl" wird erfindungsgemäß ein linearer oder verzweigter C₂₋₁₂-Alkinylrest, welcher mindestens eine Dreifachbindung aufweist, beispielsweise Ethinyl, 1-Propinyl und Propargyl, verstanden. Bevorzugt hiervon sind C₂₋₆-Alkinylreste und besonders bevorzugt sind C₃₋₄-Alkinylreste. Der Alkinylrest kann dabei auch mindestens eine Doppelbindung aufweisen.

Unter dem Begriff "Cycloalkyl" wird erfindungsgemäß ein C₃₋₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃₋₆-Cycloalkylreste.

Unter dem Begriff "Aryl" wird erfindungsgemäß ein aromatischer Rest mit 6 bis 14 Kohlenstoffatomen, vorzugsweise Phenyl, verstanden.

Unter dem Begriff "Arylalkyl" wird eine Kombination von erfindungsgemäß definierten Resten "Aryl" und "Alkyl" verstanden, wobei der Rest im Allgemeinen über die Alkylgruppe gebunden wird. Beispiele hierfür sind Benzyl, Phenylethyl oder α-Methylbenzyl, wobei Benzyl besonders bevorzugt ist.

Im Rahmen der vorliegenden Erfindung werden durch Halogen substituierte Reste, beispielsweise Halogenalkyl, einfach oder mehrfach bis zur maximal möglichen Substituentenzahl halogenierte Reste verstanden. Bei mehrfacher Halogenierung können die Halogenatome gleich oder verschieden sein. Halogen steht dabei für Fluor, Chlor, Brom oder Iod, insbesondere für Fluor, Chlor oder Brom.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die vorgenannte Bedeutung aufweist.

Gegebenenfalls substituierte Reste können einfach oder mehrfach substituiert sein, wobei bei einer Mehrfachsubstitution die Substituenten gleich oder verschieden sein können.

Die vorliegende Erfindung wird anhand der nachfolgenden Beispiele näher erläutert, ohne die Erfindung auf diese einzuschränken.

### Herstellungsbeispiele:

### Herstellung von 4-(Dimethylamino)furan-2(5H)-on

2 g (0.18 mol) 4-(Methylamino)furan-2(5H)-on werden in 20 ml 1,2-Dimethoxyethan vorgelegt und mit 0,72 g Natriumhydroxyd versetzt. Zu dieser Suspension werden 2,2 g Dimethylsulfat in 5 ml 1,2-Dimethoxyethan zudosiert und bei 40°C 5 h gerührt. Das Lösungsmittel wird im Vakuum entfernt und der Rückstand mit 50 ml Wasser versetzt. Diese Mischung wird anschließend mit 50 ml Dichlormethan extrahiert. Die organische Phase wird über Natriumsulfat getrocknet und anschließend im Vakuum eingeengt. Man erhält 1,2 g 4-(Dimethylamino)furan-2(5H)-on als Feststoff mit einer Reinheit von 96% (Ausbeute 51 %).
¹H-NMR (CDCl₃, 298K) δ: 2,93 (s, 6H), 4,59 (s, 1H), 4,69 (s, 2H)

### Beispiel 1

Zu einer Suspension von 4,1 g 4-(Dimethylamino)furan-2(5H)-on und 5 g N-[(6-Chlorpyridin-3-yl]methyl)-2,2-difluorethylamin in 50 ml Butyronitril werden bei Raumtemperatur 3,9 g Kaliumhydrogensulfat zugesetzt. Die Mischung wird 8 h auf Rückfluß erhitzt. Anschließend wird auf Raumtemperatur abgekühlt, zweimal mit 50 ml Wasser gewaschen. Das Lösungsmittel wird im Vakuum entfernt. Man erhält 6 g 4-[[(6-Chlorpyridin-3-yl)methyl](2,2-difluorethyl)amino]furan-2(5H)-on mit einer Reinheit von 92 % (82 % Ausbeute).
¹H-NMR (CDCl₃, 298K) δ: 3,53 (td, 2H), 4,52 (s, 2H), 4,82 (s, 2H), 4,83 (s, 1H), 5,96 (tt, 1H), 7,37 (d, 1H), 7,55 (dd, 1H), 8,27(d, 1H)

## Patentansprüche

1. Verfahren zur Herstellung von 4-Aminobut-2-enolid-Verbindungen der Formel (I) 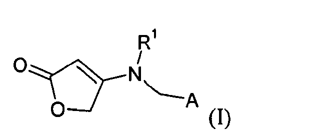 umfassend das Umsetzen einer 4-(Methylamino)furan-2(5H)-on-Verbindung der Formel (II) 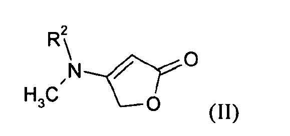 mit einem Amin der Formel (III) 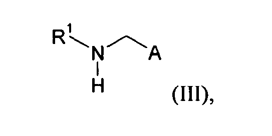 worin
R¹ für Wasserstoff, C₁₋₁₂-Alkyl, C₁₋₁₂-Halogenalkyl, C₂₋₁₂-Alkenyl, C₂₋₁₂-Halogenalkenyl, C₂₋₆-Alkinyl, C₃₋₈-Cycloalkyl, C₃₋₈-CycloalkylC₁₋₆-alkyl, C₃₋₈-Halogencycloalkyl, C₁₋₁₂-Alkoxy, C₁₋₆-AlkyloxyC₁₋₆-alkyl, C₃₋₈-HalogencycloalkylC₁₋₆-alkyl oder ArylC₁₋₆-alkyl steht;
R² für C₁₋₁₂-Alkyl, Aryl oder ArylC₁₋₆-alkyl steht; und
A für Pyrid-2-yl, Pyrid-4-yl oder Pyrid-3-yl steht, welches gegebenenfalls in 6-Position durch F, Cl, Br, CH₃, CF₃, oder OCF₃ substituiert ist, für Pyridazin-3-yl steht, welches gegebenenfalls in 6-Position durch Cl oder CH₃ substituiert ist, für Pyrazin-3-yl, 2-Chlor-pyrazin-5-yl oder für gegebenenfalls in 2-Position durch Cl oder CH₃ substituiertes 1,3-Thiazol-5-yl, für Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, das gegebenenfalls durch F, Cl, Br, CN, NO₂, C₁₋₄-Alkyl, C₁₋₃-Alkylthio, C₁₋₃-Alkylsulfonyl substituiert ist wobei jeder der Reste C₁₋₄-Alkyl, C₁₋₃-Alkylthio und C₁₋₃-Alkylsulfonyl durch F und/oder Chlor substituiert sein können, oder für ein substituiertes Heterocyclyl der folgenden Formel steht 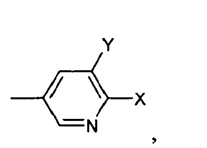
in der
X für Halogen, C₁₋₁₂-Alkyl oder C₁₋₁₂-Halogenalkyl steht, und
Y für Halogen, C₁₋₁₂-Alkyl, C₁₋₁₂-Halogenalkyl, C₁₋₁₂-Halogenalkoxy, Azido oder CN steht;
gegebenenfalls in Gegenwart einer Brønstedt-Säure.

2. Verfahren nach Anspruch 1, worin in Verbindung (III)
R¹ für C₁₋₆-Alkyl, C₁₋₆-Halogenalkyl, C₂₋₆-Alkenyl, C₂₋₆-Halogenalkenyl, C₂₋₆-Alkinyl, C₃₋₈-Cycloalkyl, C₃₋₈-CycloalkylC₁₋₆-alkyl, C₃₋₈-Halogencycloalkyl, C₃₋₈-HalogencycloalkylC₁₋₆-alkyl oder C₁₋₆-AlkyloxyC₁₋₆-alkyl steht; und
A ausgewählt ist unter 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl oder 5-Difluormethyl-6-iod-pyrid-3-yl.

3. Verfahren nach Anspruch 1 oder 2, worin die Brønstedt-Säure ausgewählt ist aus der Gruppe bestehend aus Phosphorsäure, Schwefelsäure, Salzsäure, Bromwasserstoffsäure, Flusssäure, Kaliumhydrogensulfat, Trifluoressigsäure, Essigsäure, Methansulfonsäure und p-Toluolsulfonsäure.

4. Verfahren nach einem der Ansprüche 1 bis 3, worin das molare Verhältnis der Verbindung der Formel (II) zu dem eingesetzten Amin der Formel (III) 1: 0,5 bis 1: 10 beträgt.

5. Verfahren nach einem der Ansprüche 1 bis 4, worin das Verhältnis der eingesetzten Brønstedt-Säure zu dem Amin der Formel (III) im Bereich von etwa 5 : 0,8 bis etwa 1: 1,5 liegt.

## Claims

1. Process for the preparation of 4-aminobut-2-enolide compounds of the formula (I) 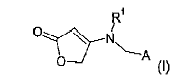 comprising the reaction of a 4-(methylamino)furan-2(5H)-one compound of the formula (II) 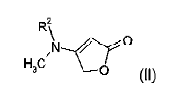 with an amine of the formula (III) 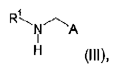 in which
R¹ represents hydrogen, C₁₋₁₂-alkyl, C₁₋₁₂-haloalkyl, C₂₋₁₂-alkenyl, C₂₋₁₂-haloalkenyl, C₂₋₆-alkynyl, C₃₋₈-cycloalkyl, C₃₋₈-cycloalkyl-C₁₋₆-alkyl, C₃₋₈-halocycloalkyl, C₁₋₁₂-alkoxy, C₁₋₈-alkoxy-C₁₋₆-alkyl, C₃₋₈-halocycloalkyl-C₁₋₆-alkyl or aryl-C₁₋₆-alkyl;
R² represents C₁₋₁₂-alkyl, aryl or aryl-C₁₋₆-alkyl; and
A represents pyrid-2-yl, pyrid-4-yl or pyrid-3-yl, which is optionally substituted in the 6-position by F, Cl, Br, CH₃, CF₃, or OCF₃, or represents pyridazin-3-yl which is optionally substituted in the 6-position by Cl or CH₃, or represents pyrazin-3-yl, 2-chicropyrazin-5-yl or represents 1,3-thiazol-5-yl optionally substituted in the 2-position by Cl or CH₃, or represents pyrimidinyl, pyrazolyl, thiophenyl, oxazolyl, isoxazolyl, 1,2,4-oxadiazolyl, isothiazolyl, 1,2,4-triazolyl or 1,2,5-thiadiazolyl, which is optionally substituted by F, Cl, Br, CN, NO₂, C₁₋₄-alkyl, C₁₋₃-alkylthio or C₁₋₃-alkylsulphonyl, where each of the radicals C₁₋₄-alkyl, C₁₋₃-alkylthio and C₁₋₃-alkylsulphonyl may be substituted by F and/or chlorine, or represents a substituted heterocyclyl of the following formula 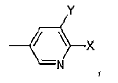
in which
X represents halogen, C₁₋₁₂-alkyl or C₁₋₁₂-haloalkyl and
Y represents halogen, C₁₋₁₂-alkyl, C₁₋₁₂-haloalkyl, C₁₋₁₂-haloalkoxy, azido or CN;
optionally in the presence of a Brønsted acid.

2. Process according to Claim 1, wherein, in compound (III),
R¹ represents C₁₋₆-alkyl, C₁₋₆-haloalkyl, C₂₋₆-alkenyl, C₂₋₆-haloalkenyl, C₂₋₆-alkynyl, C₃₋₈-cycloalkyl, C₃₋₈-cycloolkyl-C₁₋₆-alkyl, C₃₋₈-halocycloalkyl, C₃₋₈-halocycloalkyl-C₁₋₆-alkyl or C₁₋-alkoxy-C₁₋₆-alkyl; and
A is selected from 6-fluoropyrid-3-yl, 6-chloropyrid-3-yl, 6-bromopyrid-3-yl, 6-methylpyrid-3-yl, 6-trifluoromethylpyrid-3-yl, 6-trifluoromethoxypyrid-3-yl, 6-chloro-1,4-pyridazin-3-yl, 6-methyl-1,4-pyridazin-3-yl, 2-chloro-1,3-thiazol-5-yl or 2-methyl-1,3-thiazol-5-yl, 2-chloropyrimidin-5-yl, 2-trifluoromethylpyrimidin-5-yl, 5,6-difluoropyrid-3-yl, 5-chloro-6-fluoropyrid-3-yl, 5-bromo-6-fluoropyrid-3-yl, 5-iodo-6-fluoropyrid-3-yl, 5-fluoro-6-chloropyrid-3-yl, 5,6-dichloropyrid-3-yl, 5-bromo-6-chloropyrid-3-yl, 5-iodo-6-chloropyrid-3-yl, 5-fluoro-6-bromopyrid-3-yl, 5-chloro-6-bromopyrid-3-yl, 5,6-dibromopyrid-3-yl, 5-fluoro-6-iodopyrid-3-yl, 5-chloro-6-iodopyrid-3-yl, 5-bromo-6-iodopyrid-3-yl, 5-methyl-6-fluoropyrid-3-yl, 5-methyl-6-chloropyrid-3-yl, 5-methyl-6-bromopyrid-3-yl, 5-methyl-6-iodopyrid-3-yl, 5-difluoromethyl-6-fluoropyrid-3-yl, 5-difluoromethyl-6-chloropyrid-3-yl, 5-difluoromethyl-6-bromopyrid-3-yl or 5-difluoromethyl-6-iodopyrid-3-yl.

3. Process according to Claim 1 or 2, wherein the Brønsted acid is selected from the group consisting of phosphoric acid, sulphuric acid, hydrochloric acid, hydrobromic acid, hydrofluoric acid, potassium hydrogen sulphate, trifluoroacetic acid, acetic acid, methanesulphonic acid and p-toluenesulphonic acid.

4. Process according to any of Claims 1 to 3, wherein the molar ratio of the compound of the formula (II) to the amine of the formula (III) which is used is 1:0.5 to 1:10.

5. Process according to any of Claims 1 to 4, wherein the ratio of the Brønsted acid used to the amine of the formula (III) is in the range of about 5:0.8 to about 1:1.5.

## Revendications

1. Procédé pour la préparation de composés 4-aminobut-2-énolide de formule (I) 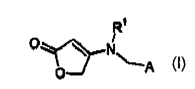 comprenant la mise en réaction d'un composé 4-(méthylamino)furan-2(5H)-one de formule (II) 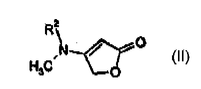 avec une amine de formule (III) 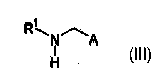 formules dans lesquelles
R¹ représente un atome d'hydrogène, un groupe alkyle en C₁-C₁₂, halogénoalkyle en C₁-C₁₂, alcényle en C₂-C₁₂, halogénoalcényle en C₂-C₁₂, alcynyle en C₂-C₈, cycloalkyle en C₃-C₆, cycloalkyl(C₃-C₈)-alkyle(C₁-C₆), halogénocycloalkyle en C₃-C₆, alcoxy en C₁-C₁₂, alkyloxy(C₁-C₆)alkyle(C₁-C₆), halogénocycloalkyl(C₃-C₈)alkyle(C₁-C₆) ou aryl-alkyle(C₁-C₆) ;
R² représente un groupe alkyle en C₁-C₁₂, aryle ou aryl-alkyle(C₁-C₆) ; et
A représente un groupe pyrid-2-yle, pyrid-4-yle ou pyrid-3-yle, qui est éventuellement substitué en position 6 par F, Cl, Br, CH₃, CF₃ ou OCF₃, représente un groupe pyridazin-3-yle qui est éventuellement substitué en position 6 par Cl ou CH₃, représente un groupe pyrazin-3-yle, 2-chloro-pyrazin-5-yle ou un groupe 1,3-thiazol-5-yle éventuellement substitué en position 2 par Cl ou CH₃, un groupe pyrimidinyle, pyrazolyle, thiophényle, oxazolyle, isoxazolyle, 1,2,4-oxadiazolyle, isothiazolyle, 1,2,4-triazolyle ou 1,2,5-thiadiazolyle, qui est éventuellement substitué par F, Cl, Br, CN, NO₂, alkyle en C₁-C₄, alkyl(C₁-C₃)thio, alkyl(C₁-C₃)sulfonyle, chacun des radicaux alkyle en C₁-C₄, alkyl(C₁-C₃)thio et alkyl(C₁-C₃)sulfonyle pouvant être substitué par F et/ou chloro, ou représente un groupe hétérocyclyle substitué de formule suivante 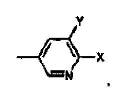
dans laquelle
X représente un atome d'halogène, un groupe alkyle en C₁-C₁₂ ou halogénoalkyle en C₁-C₁₂, et
Y représente un atome d'halogène, un groupe alkyle en C₁-C₁₂, halogénoalkyle en C₁-C₁₂, halogénoalcoxy en C₁-C₁₂, azido ou CN ;
éventuellement en présence d'un acide de Brønsted.

2. Procédé selon la revendication 1, dans lequel dans le composé (III)
R¹ représente un groupe alkyle en C₁-C₆, halogénoalkyle en C₁-C₆, alcényle en C₂-C₆, halogénoalcényle en C₂-C₆, alcynyle en C₂-C₆, cycloalkyle en C₃-C₈, cycloalkyl(C₃-C₈)alkyle(C₁-C₆), halogénocycloalkyle en C₃-C₆, halogénocycloalkyl(C₃-C₈)alkyle(C₁-C₆) ou alkyloxy(C₁-C₆)alkyle(C₁-C₆) ; et
A est choisi parmi 6-fluoro-pyrid-3-yle, 6-chloro-pyrid-3-yle, 6-bromo-pyrid-3-yle, 6-méthyl-pyrid-3-yle, 6-trifluorométhyl-pyrid-3-yle, 6-trifluorométhoxypyrid-3-yle, 6-chloro-1,4-pyridazin-3-yle, 6-méthyl-1,4-pyridazin-3-yle, 2-chloro-1,3-thiazol-5-yle ou 2-méthyl-1,3-thiazol-5-yle, 2-chloropyrimidin-5-yle, 2-trifluorométhyl-pyrimidin-5-yle, 5,6-difluoro-pyrid-3-yle, 5-chloro-6-fluoro-pyrid-3-yle, 5-bromo-6-fluoro-pyrid-3-yle, 5-iodo-6-fluoro-pyrid-3-yle, 5-fluoro-6-chloro-pyrid-3-yle, 5,6-dichloro-pyrid-3-yle, 5-bromo-6-chloropyrid-3-yle, 5-iodo-6-chloro-pyrid-3-yle, 5-fluoro-6-bromo-pyrid-3-yle, 5-chloro-6-bromo-pyrid-3-yle, 5,6-dibromo-pyrid-3-yle, 5-fluoro-6-iodo-pyrid-3-yle, 5-chloro-6-iodo-pyrid-3-yle, 5-bromo-6-iodo-pyrid-3-yle, 5-méthyl-6-fluoro-pyrid-3-yle, 5-méthyl-6-chloro-pyrid-3-yle, 5-méthyl-6-bromo-pyrid-3-yle, 5-méthyl-6-iodo-pyrid-3-yle, 5-difluorométhyl-6-fluoro-pyrid-3-yle, 5-difluorométhyl-6-chloro-pyrid-3-yle, 5-difluorométhyl-6-bromo-pyrid-3-yle ou 5-difluorométhyl-6-iodo-pyrid-3-yle.

3. Procédé selon la revendication 1 ou 2, dans lequel l'acide de Brønsted est choisi dans le groupe constitué par l'acide phosphorique, l'acide sulfurique, l'acide chlorhydrique, l'acide bromhydrique, l'acide fluorhydrique, l'hydrogénosulfate de potassium, l'acide trifluoroacétique, l'acide acétique, l'acide méthanesulfonique et l'acide p-toluènesuifonique.

4. Procédé selon l'une quelconque des revendications 1 à 3, dans lequel le rapport molaire du composé de formule (II) à l'amine de formule (III) utilisée vaut de 1:0,5 à 1:10.

5. Procédé selon l'une quelconque des revendications 1 à 4, dans lequel le rapport de l'acide de Brønsted utilisé à l'amine de formule (III) se situe dans la plage allant d'environ 5:0,8 à environ 1:1,5.
